(19) Europäisches Patentamt
European Patent Office
Office européen des brevets

(11) **EP 2 291 120 B1**

(12) **EUROPÄISCHE PATENTSCHRIFT**

(45) Veröffentlichungstag und Bekanntmachung des Hinweises auf die Patenterteilung:
**14.11.2012 Patentblatt 2012/46**

(21) Anmeldenummer: **09768972.3**

(22) Anmeldetag: **23.06.2009**

(51) Int Cl.:
***A61B 5/087*** *(2006.01)*   ***A61B 5/091*** *(2006.01)*

(86) Internationale Anmeldenummer:
**PCT/EP2009/004517**

(87) Internationale Veröffentlichungsnummer:
**WO 2009/156123 (30.12.2009 Gazette 2009/53)**

(54) **SPIROMETER MIT AUSWECHSELBAREM FLUSSROHR**

SPIROMETER WITH REPLACEABLE FLOW TUBE

SPIROMÈTRE AVEC TUBE INTERCHANGEABLE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **27.06.2008   DE 102008030536**

(43) Veröffentlichungstag der Anmeldung:
**09.03.2011   Patentblatt 2011/10**

(73) Patentinhaber: **Sendsor GmbH**
**80335 München (DE)**

(72) Erfinder:
• **SCHOLZ, Alexander**
**84558 Kirchweihdach (DE)**
• **GÜL, Murat**
**80801 München (DE)**

(74) Vertreter: **Müller - Hoffmann & Partner Patentanwälte**
**Innere Wiener Strasse 17**
**81667 München (DE)**

(56) Entgegenhaltungen:
WO-A-01/56454      WO-A-97/20500
WO-A-02/071017     WO-A-03/024317
GB-A- 2 388 665    US-A- 5 518 002

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zum Messen der Lungenatmung eines Probanden mittels eines Masseflusssensors, eines Feuchtigkeitssensors, eines Temperatursensors und eines Drucksensors.

Vorrichtungen zum Messen von Lungenfunktionen sind beispielsweise Spirometer oder Pneumotachographen, mit denen ein Lungenvolumen oder eine Atemcharakteristik eines Probanden über einen Zeitverlauf ermittelt werden kann. Dabei ist ein zeitlicher Verlauf des Atemdrucks und des Atemvolumens von besonderem Interesse für eine Diagnose von bestehenden oder beginnenden Atemkrankheiten oder anderer Pathologien im Zusammenhang mit dem Atemapparat eines Probanden. Wünschenswert ist insbesondere eine Bestimmung eines zeitlich aufgelösten Volumenflusses von durch einen Probanden aus- oder eingeatmeter Luft.

Aus dem Stand der Technik sind Vorrichtungen zur Bestimmung des Atemflusses eines Probanden bekannt. So offenbart beispielsweise das Dokument GB 2 388 665 A ein Spirometer, das eine allgemein als Luftfluss ("air flow") bezeichnete Größe anhand einer Differentialdruckmessung bestimmt. Dabei wird zusätzlich auch die Temperatur gemessen. Das Dokument WO 03/024317 A2 offenbart ebenfalls eine Vorrichtung zur Messung des Luftflusses, wobei das hierfür verwendete Messrohr für einmalige Verwendung vorgesehen ist und von der Auswerteelektronik getrennt werden kann. Ein ähnliches Spirometer mit einem abnehmbaren Messrohr wird im Dokument WO 01/56454 A2 offenbart. Aus allen diesen Dokumenten geht nicht klar hervor, ob es sich bei dem Luftfluss um einen Volumen- oder einen Massefluss handelt. Die Patentschrift US 5 518 002 und die Veröffentlichungsschrift WO 97/20500 offenbaren hingegen Spirometer, die mit thermischer Anemometrie (Hitzdrahtanemometrie) einen Massefluss messen. Das Dokument WO 02/071017 A2 beschreibt wiederum ein Spirometer mit Sensoren zur Messung eines Volumenflusses, das über weitere Sensoren zur Druck- und Temperaturmessung sowie zur Messung der Aktivität des Benutzers verfügt.

Eine direkte Bestimmung des Volumenflusses einer Lungenatmung ist jedoch herkömmlicher Weise aufwändig und bedarf einer entsprechenden Sensorik, die meist anfällig für Störungen oder Verschmutzung ist. Beispielsweise kann für eine Messung des Volumenflusses in einem Spirometer ein Sensor mit einem Turbinenrad verwendet werden, der einen Volumenfluss direkt messen kann. Derartige Sensoren sind jedoch meist relativ teuer und müssen außerdem direkt in einem Luftfluss der Atemluft eines Patienten platziert sein. Dadurch sind sie aber unweigerlich einer Verschmutzung durch Speichel oder einer möglichen Kontaminierung durch Krankheitserreger ausgesetzt, so dass meist aufwändige Reinigungsmaßnahmen und regelmäßige Funktionsüberprüfungen nötig sind.

Zudem bedürfen Vorrichtungen mit Volumenflusssensoren wie auch alle herkömmlichen Spirometer und Pneumotachographen einer aufwändigen Kalibrierung, damit gemessene Werte untereinander und mit Referenzwerten vergleichbar und damit auch aussagekräftig sind.

Der Erfindung liegt die Aufgabe zugrunde, eine Vorrichtung zum Messen der Lungenatmung eines Probanden anzugeben, die weniger aufwändig zu benutzen und instandzuhalten und weniger verschmutzungsanfällig ist und gleichzeitig hygienisch und kostengünstig ist. Außerdem liegt der Erfindung die Aufgabe zugrunde, ein entsprechendes Verfahren zum Betreiben einer derartigen Vorrichtung anzugeben.

Erfindungsgemäß wird die Aufgabe durch eine Vorrichtung gemäß Patentanspruch 1 gelöst. Ein entsprechendes Verfahren ist in Anspruch 9 definiert. Vorteilhafte Weiterentwicklungen der Erfindung sind in den abhängigen Ansprüchen angegeben.

Eine erfindungsgemäße Vorrichtung dient dem Messen einer Lungenatmung eines Probanden, insbesondere dem Bestimmen eines Volumenflusses bei der Atmung, und kann deshalb auch als Spirometer bezeichnet werden. Ein Proband ist dabei insbesondere ein menschlicher Patient. Die Vorrichtung weist eine Halteeinheit sowie eine Messeinheit auf. Die Messeinheit weist ein Rohr zum Führen von Luft auf, durch welches Luft strömen bzw. fließen kann. Aus diesem Grund wird das Rohr auch als Flussrohr bezeichnet. Ein Proband kann durch das Rohr blasen oder Luft hindurch einsaugen, so dass es ihm möglich ist, durch das Rohr hindurch auszuatmen oder einzuatmen. Hierbei strömt die gesamte von dem Probanden ausgeatmete bzw. eingeatmete Luft durch das Rohr. Das Rohr kann dabei beispielsweise ein entsprechendes Mundstück aufweisen, um ein Ansetzen des Mundes des Probanden zu erleichtern.

Außerdem weist die Messeinheit einen Masseflusssensor auf, mit Hilfe dessen ein Massefluss der in dem Rohr der Messeinheit strömenden Luft messbar ist. Der Masseflusssensor, auch Massenflusssensor genannt, kann die gesamte Masse der durch das Rohr strömenden Luft sowie einen Massefluss über einen Zeitverlauf messen und ist daher vorzugsweise innerhalb des Flussrohres angeordnet. Bläst ein Proband Luft durch das Rohr der Messeinheit, ist die gesamte Masse dieser Luft sowie der Massefluss als zeitliche Ableitung der Gesamtmasse der strömenden Luft mit Hilfe des Masseflusssensors messbar. Im Gegensatz zu einem Volumenflusssensor muss ein Masseflusssensor nicht auf möglicher Weise veränderte äußere Parameter wie Luftdruck, Luftfeuchtigkeit oder Temperatur kalibriert werden, da der Massefluss unabhängig von diesen äußeren Parametern messbar ist.

Beispielsweise kann in einem Betrieb des Masseflusssensors ein Temperaturelement wie etwa ein Pt 6,8-Element verwendet werden, bei welchem ein Heizdraht auf eine vorgegebene Temperatur aufgeheizt und auf dieser Solltemperatur gehalten wird. Strömt die Luft in dem Rohr über den Heizdraht, so kühlt dieser ab und aus der zum Nachheizen des Drahtes auf die Solltemperatur nötigen Leistung ist ein zeitlich aufgelöster Massefluss der in dem Rohr strömenden Luft bestimmbar. Dabei kühlt der Heizdraht des Temperaturelements dann ab, wenn es auf einen Sollwert oberhalb der Temperatur von bei einer Messung im Rohr strömenden Luft geheizt wird. Dafür ist es vorteilhaft, die Temperatur in dem Flussrohr durch ein weiteres Temperaturelement wie beispielsweise ein Pt 1000-Element zu messen, einen festgelegten Temperaturunterschied zu beaufschlagen und die resultierende Temperatur als Solltemperatur des Temperaturelements einzustellen. Dies kann mit einer Messbrücke aus dem Temperaturelement und dem weiteren Temperaturelement realisiert werden. Beispielsweise beträgt die Temperatur der in dem Rohr strömenden Luft 37°C, was durch das weitere Temperaturelement entsprechend messbar ist. Ein festgelegter Temperaturunterschied von beispielsweise 200°C wird dazu addiert und das Temperaturelement auf einem resultierenden Sollwert von 237°C gehalten.

Die Messeinheit ist an der Halteeinheit lösbar befestigbar, wobei eine Datenverbindung zwischen der Messeinheit und der Halteeinheit herstellbar ist. Durch die Datenverbindung ist ein Datenaustausch zwischen der Messeinheit, insbesondere deren Masseflusssensors, und der Halteeinheit möglich. Dabei kann die Datenverbindung auf einer elektrischen Leitfähigkeit, z.B. über Kabel, beruhen, aber auch drahtlos, beispielsweise über elektromagnetische Induktion, Funk oder Infrarot funktionieren. Dann weist sowohl die Messeinheit als auch die Halteeinheit entsprechende Kommunikationsmittel wie elektrische Leitungen und Kontakte, Induktionskopplungen, Funksender und - empfänger bzw. Infrarotsender und -empfänger auf. Es können dabei auch mehrere Kommunikationsarten nebeneinander vorgesehen sein. Dann sind für die Mess- und die Halteeinheit mehrere entsprechende Kommunikationsmittel vorgesehen. Wichtig ist dabei, dass die Messwerte des Masseflusssensors der Messeinheit an die Halteeinheit und Messwerte oder Daten, die in der Halteeinheit bereitgestellt sein können, an die Messeinheit bzw. deren Sensor übertragbar sind.

Die lösbare Befestigbarkeit der Messeinheit an der Halteeinheit kann auf einem lösbaren mechanischen Kontakt beruhen. Die Messeinheit kann beispielsweise auf die Halteeinheit aufgesteckt, aufgeschoben oder aufgeklebt oder in die Halteeinheit eingehakt oder eingesteckt werden. Es ist auch möglich, dass ein Aufbau eines elektrischen Kontakts, beispielsweise mit Hilfe von elektrisch leitenden Steckverbindungen, auch eine lösbare Befestigung zwischen der Messeinheit und der Halteeinheit erlaubt, sodass zusätzliche rein mechanische Kontakte eingespart werden können.

Die Halteeinheit der Vorrichtung weist ferner einen Temperatursensor, einen Feuchtigkeitssensor und einen Drucksensor auf. Mit dem Feuchtigkeitssensor der Halteeinheit ist eine Luftfeuchtigkeit in der Umgebung der Halteeinheit und damit näherungsweise auch eine Luftfeuchtigkeit in der Umgebung der Messeinheit bzw. der gesamten Vorrichtung messbar. Ist die Messeinheit an der Halteeinheit befestigt, besteht eine entsprechende räumliche Nähe dieser beiden Einheiten, so dass die an dem Feuchtigkeitssensor der Halteeinheit gemessene Luftfeuchtigkeit näherungsweise der Luftfeuchtigkeit in der Umgebung der Messeinheit bzw. der gesamten Vorrichtung entspricht. Entsprechend ist auch mit dem Drucksensor der Halteeinheit ein Luftdruck in der Umgebung der Halteeinheit und damit näherungsweise auch der Messeinheit bzw. der gesamten Vorrichtung messbar. Abweichungen der Luftfeuchtigkeit oder des Luftdrucks in einer Umgebung einer Vorrichtung sind äußerst gering und für Messungen mit entsprechenden Sensoren in der Regel vernachlässigbar. Entsprechend ist auch mit dem Temperatursensor der Halteeinheit eine Temperatur in der Umgebung der Halteeinheit messbar, die im Wesentlichen der Temperatur in der Umgebung der gesamten Vorrichtung entspricht.

In der Messeinheit ist ein Masseflusssensor bereitgestellt, dessen Funktionsweise oben bereits exemplarisch beschrieben wurde. Sein Aufbau ist - selbst mit einem oder mehreren Heizelementen - wesentlich einfacher als der Aufbau eines Volumenflusssensors, der beispielsweise auf einem Turbinenrad mit beweglichen Teilen basiert. Während ein Volumenflusssensor in hohem Maße einer Verunreinigung mit Speichel aus dem Munde eines Probanden ausgesetzt ist und somit einem Wachstum oder einer Übertragung von Krankheitserregern Raum bietet, ist eine Messeinheit mit einem Masseflusssensor wesentlich weniger Verunreinigungen ausgesetzt und somit deutlich hygienischer. Außerdem ist ein Masseflusssensor in der Regel um ein Vielfaches kostengünstiger in der Herstellung und im Betrieb als ein Volumenflusssensor. Die Vorrichtung bietet somit bei einer Kostenreduktion gegenüber einer Lösung mit einem Volumenflusssensor eine erhöhte Hygiene in der Anwendung, wobei die Qualität der Messergebnisse jedoch vergleichbar ist.

Da die Messeinheit lösbar an der Halteeinheit befestigbar ist und ein Datenaustausch zwischen diesen beiden Einheiten auf- und abgebaut werden kann, ist die Messeinheit gemäß einer Ausführungsform durch weitere Messeinheiten austauschbar. Für eine Messung einer Lungenatmung eines Probanden, insbesondere eines Volumenflusses seiner Atmung mit Hilfe der Vorrichtung, können somit verschiedene Messeinheiten aufeinander folgend an der Halteeinheit befestigt werden, so dass ein Datenaustausch zwischen den Sensoren der jeweiligen Messeinheit und den Sensoren der Halteeinheit möglich ist.

Dadurch ist es insbesondere möglich, mehrere gleichartige Messeinheiten für einen Betrieb mit einer zugehörigen Halteeinheit vorzusehen, wobei eine Messeinheit nach einem bestimmungsgemäßen Gebrauch entsorgt werden kann, während die Halteeinheit weiterhin zur Aufnahme von Messeinheiten und damit auch die Vorrichtung zum Messen einer Lungenatmung eines Probanden dienen kann. Das bedeutet, dass eine Messeinheit jederzeit durch eine gleichartige Messeinheit ausgewechselt werden kann. Insbesondere aufgrund des Bereitstellens eines kostengünstigen Masseflusssensors anstatt eines teuren Volumenflusssensors in der Messeinheit sowie durch die Anordnung von weiteren zur Analyse einer Lungenatmung hilfreichen Sensoren in der Halteeinheit, also außerhalb der Messeinheit, ist eine Messeinheit kostengünstig realisierbar und somit eine Auswechselbarkeit der Messeinheit bzw. der Messeinheiten im Sinne einer Realisierung der Messeinheiten als Wegwerfartikel möglich.

Auf diese Weise ist die Anwendung der Vorrichtung gegenüber herkömmlichen Geräten zum Bestimmen einer Lungenatmung eines Probanden deutlich hygienischer. Einerseits ist der Einsatz eines Masseflusssensor statt eines Volumenflusssensors in der Messeinheit der Vorrichtung, wie oben beschrieben, bereits aufgrund des einfachen Aufbaus des Masseflusssensors wesentlich hygienischer. Die Möglichkeit des Auswechselns der Messeinheit durch eine weitere Messeinheit erhöht dabei die Hygiene nochmals deutlich. Ein Proband kann die Vorrichtung beispielsweise mit einer ersten Messeinheit einige Male benutzen. Anschließend kann ein zweiter Proband die Vorrichtung benutzen, nachdem die erste Messeinheit durch eine zweite, noch unbenutzte Messeinheit ausgetauscht wurde. Im Gegensatz zu einer herkömmlichen Vorrichtung, bei der bestenfalls ein Mundstück auswechselbar ist, können bei einer Vorrichtung mit auswechselbarer Messeinheit bei der Verwendung einer noch unbenutzten Messeinheit keine Körperflüssigkeiten oder Krankheitserreger eines vorherigen Probanden von einem Probanden aufgenommen werden. Insbesondere bei einem Einatmen durch das Flussrohr durch den Probanden besteht keine Gefahr des Einatmens von möglicher Weise gesundheitsgefährdenden Partikeln.

Eine weitere Messeinheit kann beispielsweise auch dann anstelle einer vorherigen Messeinheit zusammen mit der Halteeinheit als Vorrichtung eingesetzt werden, wenn die vorherige Messeinheit durch Krankheitserreger möglicher Weise kontaminiert oder durch eine Verschmutzung in ihrer Funktion beeinträchtigt ist. Möglich ist auch ein Auswechseln der Messeinheit, wenn an einem weiteren Probanden Messungen durchgeführt werden sollen, die nach einer Benutzung durch den vorherigen Probanden unhygienisch und damit unzumutbar ist. Ein Austausch der Messeinheit ist aber auch dann vorgesehen, wenn eine Messung mit dieser Messeinheit nicht mehr reproduzierbar oder nicht mehr aussagekräftig ist, beispielsweise wenn die Messeinheit defekt ist. Die Messeinheiten können dafür Einwegartikel sein, die einzeln abgepackt in den Umlauf kommen und nach einer oder wenigen Benutzungen entsorgt werden sollen. Da lediglich ein einfacher Masseflusssensor bzw. ein einfacher Temperatursensor in der Messeinheit bereitgestellt ist, kann die Messeinheit entsprechend günstig und somit als Wegwerfartikel hergestellt werden. Aufgrund der Positionierung der verhältnismäßig teureren Sensoren für die Luftfeuchtigkeit und den Luftdruck in der Halteeinheit und dem Bereitstellen von lediglich einem kostengünstigen Masseflusssensor und gegebenenfalls einem bis zwei kostengünstigen Temperatursensoren in einer Messeinheit, reduzieren sich die Kosten für eine Messeinheit dabei erheblich. Des Weiteren weist die Messeinheit keine Auswerteeinheit für Daten und keine weitere Elektronik auf, so dass eine strikte Kostenbegrenzung möglich ist. Gleichzeitig erhöht sich eine Lebensdauer der gesamten Vorrichtung durch stets neue Messeinheiten gegenüber herkömmlichen Geräten beträchtlich.

Gemäß einer weiteren Ausführungsform sind die Messeinheit und alle weiteren für die Vorrichtung vorgesehenen Messeinheiten in Bezug auf einen vorgegebenen Messstandard standardisiert. Dies wird häufig als Kalibrierung oder Kalibration bezeichnet und stellt sicher, dass alle Messungen, die mit den entsprechenden Messeinheiten durchgeführt werden, quasi die gleichen Resultate erzielen. Bei herkömmlichen Spirometern oder Pneumotachographen ist stets auf eine Kalibrierung zu achten, die in regelmäßigen Abständen oder bei besonderem Bedarf zu wiederholen ist, damit die entsprechenden Messergebnisse aussagekräftig bleiben und für eine Diagnose verwendet werden können. Eine derartige Kalibrierung kann ein aufwändiger Vorgang sein und ist meist nur durch geschultes Personal und unter Zuhilfenahme von speziellen Geräten durchführbar. Aus diesem Grund müssen Spirometer häufig zum Hersteller oder zu besonders geschultem und ausgerüstetem Personal geschickt werden, um dort kalibriert zu werden, wodurch ein beträchtlicher Zeitverlust mit einem entsprechenden finanziellen Aufwand entsteht. Dies liegt darin begründet, dass Spirometer mit Volumenflusssensoren stets kalibriert werden müssen, um Änderungen von äußeren Parametern, die den Volumenfluss beeinflussen können, wie etwa des Luftdrucks, der Luftfeuchtigkeit oder der Temperatur, zu korrigieren. Um einen genauen Messwert zu erhalten muss daher mit herkömmlichen Spirometern vor jeder Messung eine Kalibrierung auf die aktuell vorherrschenden Umweltbedingungen bzw. die äußeren Parameter vorgenommen werden. Eine Vorrichtung, mit welcher eine derartige Kalibrierung durchführbar ist, ist jedoch sehr groß und somit für mobile Anwendungen unbrauchbar. Außerdem sind derartige Vorrichtungen zum Kalibrieren insbesondere für private Anwender in der Regel zu teuer.

Diese kosten- und zeitintensive Kalibrierung entfällt jedoch für eine Vorrichtung gemäß dieser Ausführungsform, da mehrere auswechselbare Messeinrichtungen jeweils bereits in einem kalibrierten Zustand bzw. genauer gesagt kalibrierungsfrei einem Benutzer bereitgestellt werden und in einem unbenutzten Zustand die entsprechende Kalibrierung im Wesentlichen nicht beeinträchtigt ist. Das bedeutet, dass ein Benutzer beispielsweise eine Messeinheit an der Halteeinheit befestigt hat, während er weitere einsatzbereite Messeinheiten lagert, um diese bei Bedarf einzusetzen. So kann beispielsweise eine erste Messeinheit an der Halteeinheit befestigt werden, um damit Messungen an einer Lungenatmung eines Probanden durchzuführen. Ist die Messeinheit aufgrund zu häufiger Benutzung oder aufgrund einer Verschmutzung nicht mehr in einem kalibrierten Zustand, so kann die Messeinheit einfach von der Halteeinheit abmontiert werden und eine weitere, neue bzw. noch unbenutzte und somit kalibrierte Messeinheit an der Halteeinheit befestigt werden. Dann liegt wieder eine kalibrierte Messeinheit bzw. eine kalibrierte Vorrichtung vor. Dieser Vorgang des Auswechselns der Messeinheit kann selbstverständlich sehr schnell erfolgen und ist selbst von einer ungeschulten Kraft oder einem Probanden selbst durchführbar.

Die Vorrichtung bietet somit durch die Auswechselbarkeit der Messeinheit eine Möglichkeit für eine hygienische Benutzung für einen oder mehrere Benutzer. Darüber hinaus liegt mit jeder neuen an der Halteeinheit befestigten Messeinheit eine neu kalibrierte Vorrichtung vor, so dass stets Messergebnisse mit einer hohen Genauigkeit erzielbar sind, ohne die komplette Vorrichtung kalibrieren zu müssen.

Gemäß einer weiteren Ausführungsform sind die Messeinheit und die weiteren Messeinheiten in Bezug auf ein Lungenvolumen eines Probanden oder auf einen festgelegten Massefluss standardisiert. Das bedeutet, dass eine Kalibrierung nicht nur anhand von allgemeinen Standardwerten wie den Dimensionen der Messeinheit oder den Charakteristiken der Sensoren der Vorrichtung, sondern individuell für typische organische Parameter jedes Probanden möglich ist. Somit kann beispielsweise für eine Messung an einem ersten Probanden eine erste Messeinheit eingesetzt werden, die auf das Lungenvolumen oder einen festgelegten Massefluss des ersten Probanden kalibriert ist und anschließend für eine zweite Messung an einem zweiten Probanden eine zweite Messeinheit nach Abmontieren der ersten Messeinheit an der Halteeinheit befestigt werden, wobei die zweite Messeinheit für den zweiten Patienten individuell kalibriert ist.

Eine individuelle Kalibrierung kann dabei nicht unbedingt äußere Parameter betreffen, sondern beispielsweise ein zu erwartendes Lungenvolumen oder ein zu erwartender Maximaldruck beim Ausatmen berücksichtigen. So kann beispielsweise eine erste Messeinheit für einen erwachsenen Probanden mit einem großen Lungenvolumen von 4 1 und einem Atemdruck von 150 mbar kalibriert sein, während eine zweite Messeinheit für ein Kind mit einem kleinen Lungenvolumen von 2 1 und einem niedrigeren Atemdruck von 40 mbar kalibriert ist.

Gemäß einer Ausführungsform weist die Halteeinheit eine Auswerteeinheit auf, an welche der Masseflusssensor der Messeinheit von ihm erfasste Messdaten übertragen kann. Die Auswerteeinheit kann anhand der empfangenen Messwerte entsprechend einen Massefluss oder einen Volumenfluss eines Ein- oder Ausatemvorgangs oder jeweils eine entsprechende zeitliche Auflösung davon oder weitere Charakteristiken der Lungenatmung eines Probanden bestimmen. Eine Auswertung der Messwerte kann dabei beispielsweise eine Bestimmung eines Volumenflusses einer Lungenatmung eines Probanden sein.

Die Auswerteeinheit ist dafür vorgesehen, durch den Feuchtigkeitssensor, den Temperatursensor, den Drucksensor oder den Masseflusssensor gemessene Messdaten zu erfassen, auszuwerten oder an weitere Einheiten weiterzuleiten. Selbstverständlich sind alle diese Messdaten einzeln oder zusammen von der Auswerteeinheit erfassbar, auswertbar oder weiterleitbar. Die Auswerteeinheit kann die ausgewerteten Daten an weitere Einrichtungen senden, beispielsweise per Funkbasierter, optischer, akustischer oder elektronischer bzw. elektrischer Datenübertragung.

Durch eine Auswertung der Messdaten aus den Sensoren der Vorrichtung ist beispielsweise eine Bestimmung eines Volumenflusses möglich, ohne dass eine direkte Messung eines Volumenflusses erforderlich ist. Hierzu ist insbesondere aus den Messdaten des Temperatursensors, des Drucksensors, des Feuchtigkeitssensors und des Masseflusssensors ein Volumenfluss über einen Zeitverlauf bestimmbar. Die von diesen Sensoren gemessenen Messwerte, also die Masseflusswerte für die in dem Rohr strömende Luft sowie die Messwerte der Lufttemperatur, der Luftfeuchtigkeit und des Luftdrucks in der Umgebung der Vorrichtung können von der Auswerteeinheit zusammen ausgewertet werden, um einen entsprechenden Volumenfluss zu ermitteln. Ein Volumenfluss der Luft in dem Flussrohr wird somit nicht direkt gemessen, sondern indirekt aus den Messwerten der Sensoren der Vorrichtung bestimmt.

Es wird dabei jedoch keineswegs nur ein Volumenflusssensor durch eine Vielzahl von Sensoren zum indirekten Messen eines Volumenflusses ausgetauscht. Vielmehr werden unterschiedliche Sensortypen auch an unterschiedlichen Positionen in der Vorrichtung bereitgestellt, die es erlauben, mithilfe einer Annahme einer weitgehenden Ähnlichkeit der äußeren Parameter wie Luftdruck der Umgebung, Luftfeuchtigkeit und Umgebungstemperatur mit den Parametern in der unmittelbaren Umgebung eines Masseflusssensors eine räumliche Trennung der Sensoren für eine Volumenflussbestimmung zu erreichen, welche zahlreiche Vorteile mit sich bringt. Der einer Atemluft auszusetzende Masseflusssensor ist in dem Rohr angeordnet, während die relativ langlebigen, jedoch auch teuren Sensoren zum Bestimmen der Luftfeuchtigkeit, des Luftdrucks und der Temperatur in der Halteeinheit angeordnet sind. Somit ist nicht nur ein kalibrierungsfreies Bedienen der Vorrichtung möglich, sondern das Rohr samt Masseflusssensor ist darüber hinaus auswechselbar, so dass eine größtmögliche Hygiene bei einem kleinstmöglichen Betriebs- und Kostenaufwand möglich ist.

Die Beziehung zwischen dem mit Hilfe des Masseflusssensors ermittelten Massefluss m als zeitliche Ableitung der Masse der in dem Rohr strömenden Luft und dem zu bestimmenden Volumenfluss V als zeitliche Ableitung des Volumens der in dem Rohr strömenden Luft lässt sich folgendermaßen darstellen:

$$\dot{m} = \dot{V} \cdot \rho , \qquad (1)$$

wobei für $m = \int \dot{m}\dfrac{d}{dt}$ und $V = \int \dot{V}\dfrac{d}{dt}$ gelten und wobei für den Druck r gilt:

$$\rho = \frac{1}{(T/°C + 273.16)}\left(\frac{p - \varphi \cdot p_S(T)}{R_L} + \frac{\varphi \cdot p_S(T)}{R_D}\right). \quad (2)$$

Der Massefluss m entspricht demnach dem Produkt aus dem Volumenfluss V und dem Druck r. Die Beziehung für den Druck r berücksichtigt eine Umrechnung der Temperatur von Grad Celsius in Kelvin sowie die spezifischen Gaskonstanten $R_L$ für trockene Luft von $R_L$ = 287,05 J/kgK und $R_D$ für Wasserdampf von $R_D$= 461 J /kgK für die feuchte Luft in dem Rohr.

Die Luftfeuchtigkeit j in der Atemluft, welche durch das Rohr strömt, wird beim Ausatmen als maximal angenommen, sodass diese Luft also die Luftfeuchtigkeit von Wasserdampf aufweist. Wasserdampf selbst hat einen Sättigungsdampfdruck $p_s$, der abhängig von der Temperatur der Atemluft ist, wodurch sich zusammen mit der Luftfeuchtigkeit der Atemluft j aus dem Feuchtigkeitssensor der Wasserdampfdruck $p_D$ gemäß folgender Beziehung ergibt:

$$p_D = j \cdot p_s \quad (3)$$

Die Zusammensetzung für die Dichte $\rho$ ergibt sich aus der Summe der Dichte von trockener Luft $\rho_L$ und der Dichte von Wasserdampf $\rho_D$ und damit zu

$$\rho = \rho_L + \rho_D. \quad (4)$$

Zusammen mit der Gaskonstante für trockene Luft $R_L$ von 287,05 J /kgK und der Gaskonstante für Wasserdampf $R_D$ 461 J/kgK folgt für die Gaskonstante $R_F$ der feuchten Luft in dem Rohr:

$$R_F = R_L /(1 - (\varphi \cdot p_S/p) \cdot (1 - R_L/R_D)) \quad (5)$$

Aus der nach DIN 4108 auf 0,1% genauen sogenannten Magnus-Formel für den Sättigungswasserdampfdruck $p_s$ ergibt sich die Beziehung

$$p_S(T) = p_0 \cdot \exp((17,62 \cdot T)/(243,12°C + T)), \quad (6)$$

wobei $p_0$ = 611,2 Pa und T = Temperatur in °C der im Rohr strömenden Luft ist.

Schließlich lässt sich aus den obigen Gleichungen (1) bis (6) der Volumenfluss V aus

$$\dot{V} = \frac{\dot{m} \cdot (T/°C + 273,16)K * R_L}{p \cdot \left(1 - \left(\varphi \cdot \dfrac{p_0 \cdot \exp\left(\dfrac{17,62 * T}{243,12°C + T}\right)}{p}\right) \cdot \left(1 - \dfrac{R_L}{R_D}\right)\right)} \quad (7)$$

bestimmen. Auf diese Weise ist eine indirekte Bestimmung des Volumenflusses V der in dem Rohr der Messeinheit strömenden Luft möglich, so dass auf störanfällige und aufwändig zu betreibende Sensoren zum direkten Messen eines Volumenflusses verzichtet werden kann. Die Messeinheit der Vorrichtung weist dabei gleichzeitig einen weniger störanfälligen bzw. verschmutzbaren sowie kostengünstigeren Masseflusssensor auf, wobei dennoch ausreichend genaue Werte für einen Volumenfluss ermittelbar sind.

Diese Ermittlung des Volumenflusses V aus den Messwerten der Sensoren der Vorrichtung und aus den festgelegten Annahmen über physikalische Umgebungsparameter in dem Flussrohr lässt sich in einem Verfahren realisieren, in welchem die oben aufgezeigten Messwerte von den Sensoren eingeholt werden und entsprechend den Gleichungen (1) bis (7) verarbeitet werden. Hierführ können entsprechende Einheiten zum Einholen und Verarbeiten der Messdaten vorgesehen sein.

Gemäß einer weiteren Ausführungsform ist ein Tischgerät vorgesehen, das die Auswerteeinheit aufweist. Dann ist ein Bereitstellen einer Auswerteeinheit in der Halteeinheit nicht zwingend erforderlich. Eine Datenkommunikation zwischen dem Sensor der Messeinheit und den Sensoren der Halteeinheit mit der Auswerteeinheit ist jedoch vorgesehen. Diese Kommunikation kann beispielsweise per Funk, Infrarot, elektrischem Strom über Kabel oder elektromagnetischer Induktion erfolgen und erlaubt der Auswerteeinheit, auf die Messdaten der Sensoren zuzugreifen, um diese auszuwerten.

Gemäß einer weiteren Ausführungsform ist durch die Auswerteeinheit eine Anzahl von Benutzungszyklen der Messeinheit erfassbar. Die Auswerteeinheit kann dabei beispielsweise in der Halteeinheit oder in einem Tischgerät vorgesehen sein und zählen, wie oft eine Messeinheit verwendet wurde. Somit kann ein Erreichen einer festgelegten Obergrenze für die Benutzungszyklen einer Messeinheit anhand eines Vergleichs zwischen dem entsprechenden festgelegten Wert gemäß der Obergrenze und dem Zählerstand gemäß der Anzahl der getätigten Benutzungen bestimmt werden.

Gemäß einer weiteren Ausführungsform weist die Messeinheit eine Speichereinheit zum Speichern der von der Auswerteeinheit erfassten, ausgewerteten oder weitergeleiteten Messdaten oder der Anzahl von Benutzungszyklen der Vorrichtung auf. Somit sind die von der Auswerteeinheit ermittelten Zählerstände speicherbar. Vorzugsweise werden unterschiedliche Messeinrichtungen durch einen entsprechenden Identifizierungscode identifiziert, so dass bei einem Wechsel der Messeinheit ein neuer Zählerstand der Benutzungszyklen der neuen Messeinheit zugeordnet werden kann. Eine Identifizierung kann dabei aufgrund einer Datenverbindung zwischen der Messeinheit und der Halteeinheit erfolgen, dementsprechend also aufgrund eines elektrischen Kontaktes über Kabel oder berührungslos ablaufen. Die Identifizierung erfolgt dabei automatisch bei einem Befestigen der entsprechenden Messeinrichtung an der Halteeinrichtung oder aufgrund einer Anweisung durch einen Benutzer, beispielsweise durch eine Bedienung einer entsprechenden Einheit. Falls eine zuvor bereits benutzte Messeinheit erneut an der Halteeinheit befestigt wird, kann die Auswerteeinheit diese Messeinheit identifizieren und ihr einen zuvor ermittelten Zählerstand für die Benutzungszyklen zuordnen. Somit wird sichergestellt, dass trotz einer Auswechselbarkeit der Messeinheiten keine Messeinheit öfter verwendet wird, als mit Hilfe eines entsprechenden voreingestellten Referenzwerts festgelegt ist.

Gemäß einer weiteren Ausführungsform ist durch die Vorrichtung ein Warnsignal ausgebbar, wenn eine voreingestellte Anzahl von Benutzungszyklen der Messeinheit erreicht ist. Dies dient dazu, einen Benutzer auf das Erreichen einer Obergrenze an Benutzungszyklen aufmerksam zu machen und ihn zu veranlassen, die Messeinheit durch eine weitere Messeinheit auszuwechseln. Es ist auch möglich, dass bei einem Erreichen der voreingestellten Anzahl an Benutzungszyklen die Vorrichtung eine Benutzung solange verhindert, bis die Messeinheit ausgetauscht wird.

Gemäß einem Verfahren zum Betreiben einer Vorrichtung zum Messen der Lungenatmung eines Probanden mit einer Messeinheit und einer Halteeinheit, wird eine Messeinheit sowie eine weitere Messeinheit zum Messen eines Volumenflusses oder eines Masseflusses von Luft kalibriert, so dass mit beiden Messeinheiten im Wesentlichen gleiche Messwerte erfassbar sind. Die Messeinheit wird an der Halteeinheit lösbar befestigt. Anschließend wird die Messeinheit durch die ka-librierte weitere Messeinheit ausgewechselt, wenn die Messeinheit nicht mehr kalibriert ist.

Gemäß einer weiteren Ausführungsform erfolgt zwischen dem Schritt des lösbaren Verbindens und des Auswechselns ein Erfassen einer Anzahl von Benutzungszyklen der Messeinheit durch die Auswerteeinheit. Ferner wird ein Warnsignals beim Erreichen einer voreingestellten Anzahl von Benutzungszyklen der Messeinheit der Vorrichtung ausgegeben.

Diese und weitere Vorteile und Merkmale der Erfindung werden nachfolgend anhand von Beispielen unter Zuhilfenahme der begleitenden Figuren näher erläutert. Es zeigen:

Figur 1 eine in skizzierter Form dargestellte Vorrichtung gemäß einer ersten Ausführungsform der Erfindung; und

Figur 2 eine in skizzierter Form dargestellte Vorrichtung gemäß der ersten Ausführungsform der Erfindung.

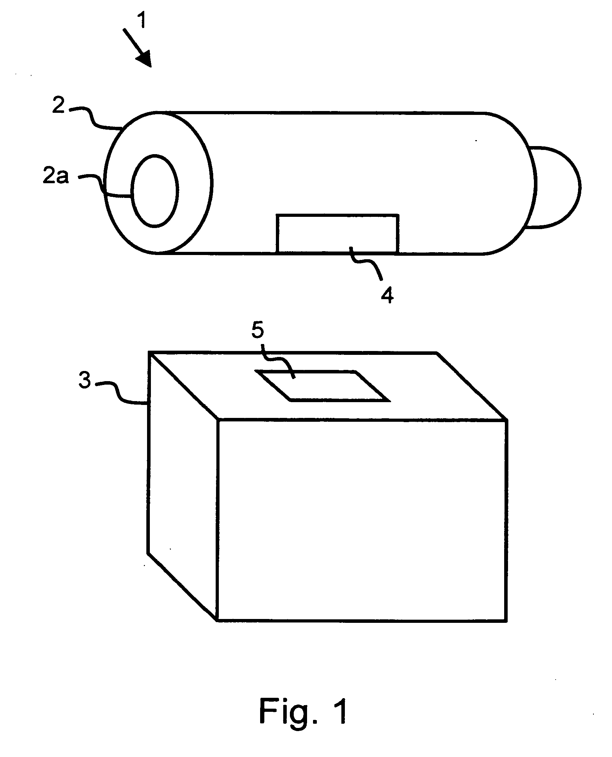

Fig. 1 zeigt eine Skizze einer Vorrichtung 1 zum Messen einer Lungenatmung eines Probanden. Die Vorrichtung 1 weist eine Messeinheit 2 mit einem Rohr 2a sowie eine Halteeinheit 3 auf, wobei die Messeinheit 2 an der Halteeinheit 3 lösbar befestigbar ist. Gezeigt ist eine Anordnung, in der die Messeinheit 2 nicht an der Halteeinheit 3 befestigt ist.

Die Messeinheit 2 ist an der Halteeinheit 3 mit Hilfe von Verbindungseinheiten 4, 5 befestigbar. Dabei weist die Messeinheit 2 die erste Verbindungseinheit 4 und die Halteeinheit 5 die zweite Verbindungseinheit 5 auf. Mit Hilfe der Verbindungseinheiten 4, 5 ist sowohl eine Befestigung der Messeinheit 2 an der Halteeinheit 3 möglich, als auch das Herstellen eines Kontaktes, mit Hilfe dessen ein Datenaustausch zwischen der Messeinheit 2 und der Halteeinheit 3 möglich ist. Dazu können die Verbindungseinheiten 4, 5 beispielsweise elektrische Kontakte aufweisen.

Durch das Rohr 2a der Messeinheit 2 kann Luft fließen bzw. strömen, welche beispielsweise von einem Probanden beim Ausatmen hinein geblasen wird, weshalb es auch als Flussrohr 2a bezeichnet wird. Die Vorrichtung 1 dient dem Messen der Lungenatmung des Probanden, und die Messeinheit 2 nimmt zu diesem Zweck Messdaten über einen Massefluss von in dem Flussrohr 2a strömender Luft auf. Wenn die gesamte Luft, die von einem Probanden ein - oder ausgeatmet wird, durch das Flussrohr 2a strömt, ist durch die Messeinheit 2 ein zeitlich aufgelöster Massefluss dieser Luft messbar, welcher für eine Charakterisierung der Lungenatmung des Probanden nutzbar ist. Mit Hilfe der Vorrichtung kann beispielsweise eine Intensität einer Lungenkontraktion eines Probanden ermittelt werden, wodurch eine Diagnose von Krankheiten ermöglicht wird.

Da die Messeinheit 2 lösbar an der Halteeinheit befestigbar ist, kann die Messeinheit 2 auf der Halteeinheit befestigt werden, so dass die Vorrichtung zum Bestimmen einer Lungenfunktion eines Probanden dient. Die Messeinheit kann auch von der Halteeinheit 3 gelöst werden und durch eine weitere, hier nicht dargestellte Messeinheit ausgewechselt werden, welche ebenso wie die Messeinheit 2 lösbar an der Halteeinheit befestigbar ist sowie ein Flussrohr aufweist und dafür vorgesehen ist, einen Massefluss von durch das Flussrohr strömender Luft zu messen. Dadurch kann die Messeinheit 2, die von einem ersten Probanden auf der Halteeinheit 3 zum Bestimmen einer Lungenatmung benutzt wurde, nach Belieben durch eine weitere Messeinheit ausgetauscht werden, insbesondere wenn ein weiterer Proband die Vorrichtung benutzen soll oder die Messeinheit 2 beschädigt, verschmutzt oder nicht mehr ausreichend kalibriert ist. Die Messeinheit 2 und alle weiteren an der Halteeinheit 3 befestigbaren Messeinheiten sind in einem unbenutzten Zustand auf einen vorgegebenen Messstandard standardisiert, also auf vorgegebene Parameter und eine vorgegebene Verwendung kalibriert. Die vorgegebenen Parameter betreffen hierbei etwa die Dimensionen des Flussrohrs wie dessen Länge und Durchmesser. Die vorgegebene Verwendung betrifft etwa ein zu erwartendes Lungenvolumen oder ein zu erwartender Atemdruck eines Probanden. Durch die Kalibrierung aller an der Halteeinheit 3 befestigbaren Messeinheiten 2 ist sichergestellt, dass eine Vorrichtung 1 mit einer an der Halteeinheit 3 befestigten, noch unbenutzten Messeinheit 2, stets reproduzierbare Messergebnisse liefert.

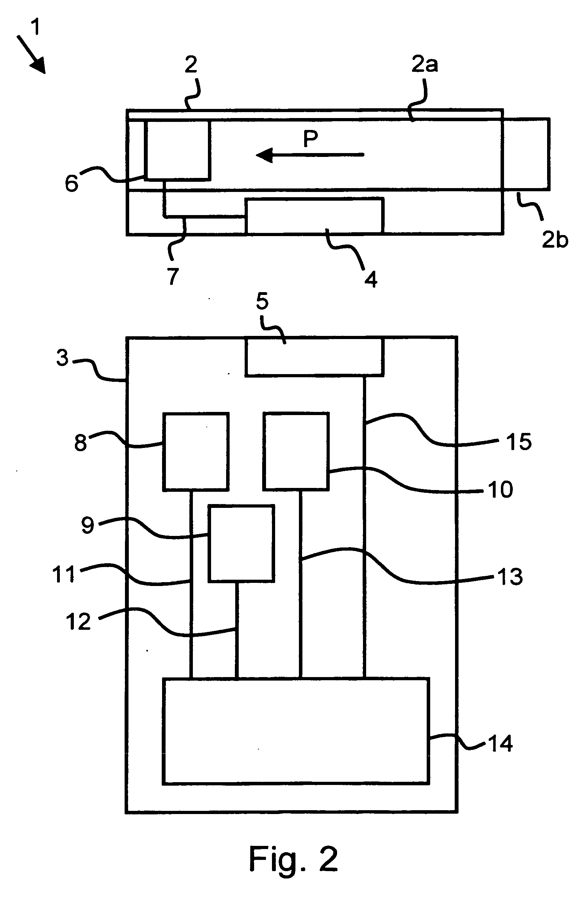

Fig. 2 zeigt eine weitere Skizze der Vorrichtung 1 aus Fig. 1. Die Messeinheit 2 weist das Flussrohr 2a auf und ist auf der Halteeinheit 3 befestigbar. Die Messeinheit 2 weist einen Masseflusssensor 6 auf, der dafür vorgesehen ist, die Masse von in der Messeinheit 2 strömender Luft zu messen. Zu diesem Zweck ist der Masseflusssensor 6 in dem Flussrohr 2a angeordnet. Luft kann durch einen Probanden durch das Flussrohr 2a geblasen oder durch das Flussrohr 2a hindurch angesaugt werden. Zu diesem Zweck weist das Flussrohr 2a ein Mundstück 2b auf, an welches ein Proband seinen Mund so ansetzen kann, dass die gesamte Luft, die sich aus seinem oder in seinen Mundraum bewegt, durch das Flussrohr 2a strömt. Die Richtung der in der Messeinheit 2 strömenden Luft bei einem Aus-atemvorgang ist durch den Pfeil P angezeigt, bei einem Einatmen strömt die Luft in dem Flussrohr 2a im Wesentlichen in die dem Pfeil P entgegen gesetzte Richtung. Der Masseflusssensor 6 ist über eine Datenverbindung 7 mit der Verbindungseinheit 4 verbunden, so dass ein Austausch der entsprechenden Messdaten des Masseflusssensors 6 und der Verbindungseinheit 4 erfolgen kann.

Die Halteeinheit 3 weist einen Temperatursensor 8, einen Feuchtigkeitssensor 9 und einen Drucksensor 10 auf. Der Temperatursensor 8 ist dafür vorgesehen, die Temperatur in seiner Umgebung zu messen. Diese Temperatur entspricht im Wesentlichen der Temperatur in der Umgebung der Halteeinheit 3 und der gesamten Vorrichtung 1. Der Feuchtigkeitssensor 9 ist dafür vorgesehen, die Luftfeuchtigkeit seiner Umgebungsluft zu messen und der Drucksensor 10 ist dafür vorgesehen, einen Luftdruck in seiner Umgebung zu messen. Der Temperatursensor 8 ist über eine Leitung 11, der Feuchtigkeitssensor 9 über eine Leitung 12 und der Drucksensor 10 über eine Leitung 13 mit einer Auswerteeinheit 14 verbunden, so dass Daten zwischen den drei Sensoren 8, 9, 10 der Halteeinheit 3 und der Auswerteeinheit 14 austauschbar sind. Die Auswerteeinheit 14 ist wiederum über eine Leitung 15 mit der Verbindungseinheit 5 der Halteeinheit 3 verbunden. Bei einem einen Datenaustausch ermöglichenden Kontakt zwischen den Verbindungseinheiten 4, 5 ist somit ein Datenaustausch zwischen dem Masseflusssensor 6, dem Temperatursensor 8, dem Feuchtigkeitssensor 9, dem Drucksensor 10 und der Auswerteeinheit 14 möglich. Die Auswerteeinheit 14 kann die Messdaten aller vier Sensoren 6, 8, 9, 10 der Vorrichtung 1 erfassen und daraus einen Volumenfluss der in der Messeinheit 2 strömenden Luft ermitteln.

Dieser Volumenfluss ist durch die Auswerteeinheit 14 nach außen an einen Benutzer der Vorrichtung 1 beispielsweise über eine drahtgebundene oder eine drahtlose Datenkommunikation ausgebbar, so dass diese Information über den Volumenfluss für eine Diagnose in Bezug auf die Lungenfunktion eines Probanden verwendbar ist. Die Messdaten aller Sensoren 6, 8, 9, 10 der Vorrichtung 1 sind somit nicht nur erfassbar und auswertbar, sondern auch nach außen oder an weitere Einheiten in der Vorrichtung, wie beispielsweise eine Anzeigeeinheit zum Anzeigen der erfassten oder ausgewerteten Messdaten, weiterleitbar. Die Auswerteeinheit 14 kann die erfassten oder ausgewerteten Daten beispielsweise auch an einen Lautsprecher weiterleiten, mit Hilfe dessen ein Warnsignal ausgebbar ist, wenn eine voreingestellte Anzahl von Benutzungszyklen der Messeinheit 2 erreicht ist. Zu diesem Zweck vergleicht die Auswerteeinheit 14 die erfasste Anzahl von Benutzungszyklen jeder an der Halteeinheit 3 befestigten Messeinheit 2 mit einem entsprechenden Vergleichswert. Entspricht die erfasste Anzahl von Benutzungszyklen für eine Messeinheit 2 der entsprechenden voreingestellten Anzahl von Benutzungszyklen, kann die Auswerteeinheit 14 eine entsprechende Information an eine Anzeigeeinheit oder einen Lautsprecher weiterleiten, so dass eine entsprechende Warnmeldung oder ein Warnsignal ausgegeben wird. Ein Benutzer der Vorrichtung 1 ist dann angehalten, die Messeinheit 2 durch eine weitere Messeinheit auszutauschen.

Für eine Benutzung der Vorrichtung 1 befestigt ein Benutzer die Messeinheit 2 an der Halteeinheit 3, wodurch eine Datenkommunikation zwischen der Messeinheit und der Halteeinheit 3 über die Verbindungseinheiten 4, 5 möglich ist. Ein Proband setzt seinen Mund an dem Mundstück 2b des Flussrohrs 2a der Messeinheit 2 an und atmet beispielsweise durch das Flussrohr 2 aus. Dadurch strömt die gesamte Luft des Ausatmens durch das Flussrohr 2 und der darin angeordnete Masseflusssensor 6 misst eine Gesamtmasse der über ihn hinweg strömenden Luft oder einen zeitlich aufgelösten Massefluss der Luft. Zeitnah dazu messen der Temperatursensor 8, der Feuchtigkeitssensor 9 und der Drucksensor 10 die Temperatur, die Luftfeuchtigkeit bzw. den Luftdruck in der Umgebung der Vorrichtung 1. Die Auswerteeinheit 14 empfängt die Messwerte aller vier Sensoren 6, 8, 9, 10 und ermittelt daraus einen Volumenfluss der in dem Flussrohr 2a strömenden bzw. bereits geströmten Luft. Die ausgewerteten Ergebnisse können anschließend von der Auswerteeinheit an eine Speichereinheit, eine Anzeigeeinheit, einen Lautsprecher oder weitere Einheiten weitergeleitet werden, um die Ergebnisse abzuspeichern, anzuzeigen, als entsprechende akustische Signale auszugeben oder weitere Datenverarbeitungsschritte zu erlauben.

Ist die Messeinheit 2 beschädigt, durch Krankheitserreger kontaminiert, aus hygienischen Gesichtspunkten für eine weitere Benutzung unzumutbar oder erlaubt keine reproduzierbaren Messungen mehr, kann die Messeinheit 2 durch einen Benutzer von der Halteeinheit 3 gelöst werden. Da die Messeinheit 2 lösbar und einfach aufgebaut ist, kann sie einfach entsorgt werden. Anschließend wird eine weitere, noch unbenutzte Messeinheit an der Halteeinheit 3 befestigt, so dass eine saubere, kalibrierte Vorrichtung 1 zum Messen der Lungenatmung eines Probanden vorliegt.

**Patentansprüche**

1. Vorrichtung (1) zum Messen der Lungenatmung eines Probanden, mit einer Halteeinheit (3) und einer ein Rohr (2a) zum Führen von Luft aufweisenden Messeinheit (2), wobei die Messeinheit (2) einen Masseflusssensor (6) zum Messen eines Masseflusses der in dem Rohr (2a) strömenden Luft aufweist, wobei die Messeinheit (2) an der Halteeinheit (3) lösbar befestigbar ist, wobei eine Datenverbindung zwischen der Messeinheit (2) und der Halteeinheit (3) herstellbar ist, **dadurch gekennzeichnet, dass**

   die Halteeinheit (3) einen Temperatursensor (8), einen Feuchtigkeitssensor (9), einen Drucksensor ( 10), und eine Auswerteeinheit ( 14) aufweist, an welche vom Masseflusssensor (6) der Messeinheit (2) erfasste Messdaten übertragbar sind, wobei durch die Auswerteeinheit durch den Masseflusssensor (6), den Temperatursensor (8), den Feuchtigkeitssensor (9) oder den Drucksensor ( 10) gemessene Messdaten erfassbar, auswertbar oder weiterleitbar sind, und

   aus den Messdaten des Masseflusssensors (6), des Temperatursensors (8), des Drucksensors (9) und des Feuchtigkeitssensors ( 10) ein Volumenfluss über einen Zeitraum bestimmbar ist.

2. Vorrichtung (1) nach Anspruch 1, **dadurch gekennzeichnet, dass** die Messeinheit (2) durch weitere Messeinheiten austauschbar ist.

3. Vorrichtung (1) nach einem der vorangehenden Ansprüche, **dadurch gekennzeichnet, dass** die Messeinheit (2) und die weiteren Messeinheiten in Bezug auf einen vorgegebenen Messstandard standardisiert sind.

4. Vorrichtung (1) nach einem der vorangehenden Ansprüche, **dadurch gekennzeichnet, dass** die Messeinheit (2) und die weiteren Messeinheiten in Bezug auf ein Lungenvolumen eines Probanden oder auf einen festgelegten Massefluss standardisiert sind.

5. Vorrichtung (1) nach einem der vorangehenden Ansprüche, **dadurch gekennzeichnet, dass** ein Tischgerät vorgesehen ist, das die Auswerteeinheit ( 14) aufweist.

6. Vorrichtung (1) nach einem der vorangehenden Ansprüche, **dadurch gekennzeichnet, dass** durch die Auswerteeinheit ( 14) eine Anzahl von Benutzungszyklen der Messeinheit (2) erfassbar ist.

7.  Vorrichtung (1) nach einem der vorangehenden Ansprüche, **dadurch gekennzeichnet, dass** die Messeinheit (2) eine Speichereinheit zum Speichern der von der Auswerteeinheit ( 14) erfassten, ausgewerteten oder weitergeleiteten Messdaten oder der Anzahl von Benutzungszyklen der Vorrichtung ( 1 ) aufweist.

8.  Vorrichtung (1) nach einem der vorangehenden Ansprüche, **dadurch gekennzeichnet, dass** durch die Vorrichtung ( 1 ) ein Warnsignal ausgebbar ist, wenn eine voreingestellte Anzahl von Benutzungszyklen der Messeinheit (2) erreicht ist.

9.  Verfahren zum Betreiben einer Vorrichtung (1) zum Messen der Lungenatmung eines Probanden mit einer Messeinheit (2) und einer Halteeinheit (3), **gekennzeichnet durch** die folgenden Schritte:

    - Kalibrieren der Messeinheit (2) und einer weiteren Messeinheit zum Messen eines Volumenflusses oder eines Masseflusses von Luft, so dass mit beiden Messeinheiten im Wesentlichen gleiche Messwerte erfassbar sind;
    - lösbares Befestigen der Messeinheit (2) an der Halteeinheit (3);
    - Messen einer Temperatur **durch** einen Temperatursensor, einer Feuchtigkeit durch einen Feuchtigkeitssensor, eines Druckes **durch** einen Drucksensor und eines Masseflusses **durch** einen Masseflusssensor,
    - Bestimmen eines Volumenflusses aus Messdaten der Messungen des Temperatursensors, de Drucksensors, des Feuchtigkeitssensors und des Masseflusssensors; und
    - Auswechseln der Messeinheit (2) **durch** die kalibrierte weitere Messeinheit (2'), wenn die Messeinheit (2) nicht mehr kalibriert ist.

10. Verfahren nach Anspruch 9, **dadurch gekennzeichnet, dass** zwischen dem Schritt des lösbaren Verbindens und des Auswechselns die folgenden weiteren Schritte erfolgen können:

    - Erfassen einer Anzahl von Benutzungszyklen der Messeinheit (2) durch eine Auswerteeinheit ( 14); und
    - Ausgeben eines Warnsignals beim Erreichen einer voreingestellten Anzahl von Benutzungszyklen der Messeinheit (2).

## Claims

1.  Device (1) for measuring the pulmonary respiration of a subject, with a holding unit (3) and a measuring unit (2) having a tube (2a) for passage of air, wherein the measuring unit (2) has a mass flow sensor (6) for measuring a mass flow of the air flowing in the tube (2a), wherein the measuring unit (2) is removably fastenable to the holding unit (3), wherein a data connection can be made between the measuring unit (2) and the holding unit (3), **characterised in that**
    the holding unit (3) has a temperature sensor (8), a humidity sensor (9), a pressure sensor (10), and
    an evaluation unit (14) to which measurement data recorded by the mass flow sensor (6) of the measuring unit (2) can be transferred, wherein measurement data measured by the mass flow sensor (6), the temperature sensor (8), the humidity sensor (9) or the pressure sensor (10) can be recorded, evaluated or passed on, and
    a volume flow can be determined over a period of time from the measurement data of the mass flow sensor (6), the pressure sensor (10),the temperature sensor (8), and the humidity sensor (9).

2.  Device (1) in accordance with claim 1, **characterised in that** the measuring unit (2) can be replaced by further measuring units.

3.  Device (1) in accordance with one of the preceding claims, **characterised in that** the measuring unit (2) and the further measuring units are standardised in relation to a predetermined measurement standard.

4.  Device (1) in accordance with one of the preceding claims, **characterised in that** the measuring unit (2) and the further measuring units are standardised in relation to a pulmonary volume of a subject or to a defined mass flow.

5.  Device (1) in accordance with one of the preceding claims, **characterised in that** a table unit is provided which has the evaluation unit (14).

6.  Device (1) in accordance with one of the preceding claims, **characterised in that** the evaluation unit (14) can record a number of usage cycles of the measuring unit (2).

7. Device (1) in accordance with one of the preceding claims, **characterised in that** the measuring unit has a storage unit for storing the measurement data recorded, evaluated or passed on by the evaluation unit (14), or the number of usage cycles of the device (1).

8. Device (1) in accordance with one of the preceding claims, **characterised in that** a warning signal can be given by the device (1) when a preset number of usage cycles of the measuring unit (2) is reached.

9. Method for operating a device for measuring the pulmonary respiration of a subject, with a measuring unit (2) and a holding unit (3), **characterised by** the following steps:

   - calibration of the measuring unit (2) and a further measuring unit for measuring a volume flow or a mass flow of air so that essentially the same measurements can be recorded with both measuring units;
   - removable fastening of the measuring unit (2) to the holding unit (3)
   - measurement of a temperature by temperature sensor, a humidity by a humidity sensor, a pressure by a pressure sensor, and a mass flow by a mass flow sensor,
   - determining a volume flow from measurement data of the measurements of the temperature sensor, the pressure sensor, the humidity sensor and the mass flow sensor; and
   - replacing the measuring unit (2) by the calibrated further measuring unit (2'), when the measuring unit (2) is no longer calibrated.

10. Method in accordance with claim 9, **characterised in that** the following further steps can take place between the step of the removable connection and the replacement:

   - recording of a number of usage cycles of the measuring unit (2) by an evaluation unit (14); and
   - giving a warning signal when a preset number of usage cycles of measuring unit (2) is reached.

**Revendications**

1. Dispositif (1) pour mesurer la respiration pulmonaire d'un sujet à examiner, avec une unité de support (3) et une unité de mesure (2) comportant un tube (2a) pour guider l'air, étant précisé que l'unité de mesure (2) comporte un capteur de flux de masse (6) pour mesurer un flux de masse de l'air s'écoulant dans le tube (2a), que ladite unité de mesure (2) est apte à être fixée de manière amovible à l'unité de support (3), et qu'une liaison de données est apte à être réalisée entre l'unité de mesure (2) et l'unité de support (3), **caractérisé en ce que**
l'unité de support (3) comporte un capteur de température (8), un capteur d'humidité (9), un capteur de pression (10), et
une unité d'analyse (14) à laquelle les données de mesure relevées par le capteur de flux de masse (6) de l'unité de mesure (2) sont aptes à être transmises, étant précisé que grâce à l'unité d'analyse, les données de mesure mesurées par le capteur de flux de masse (6), le capteur de température (8), le capteur d'humidité (9) ou le capteur de pression (10) peuvent être relevées, analysées ou retransmises, et
qu'à partir des données de mesure du capteur de flux de masse (6), du capteur de température (8), du capteur de pression (9) et du capteur d'humidité (10), un flux de volume est apte à être défini sur un laps de temps.

2. Dispositif (1) selon la revendication 1, **caractérisé en ce que** l'unité de mesure (2) est apte à être remplacée par d'autres unités de mesure.

3. Dispositif (1) selon l'une des revendications précédentes, **caractérisé en ce que** l'unité de mesure (2) et les autres unités de mesure sont normalisées par rapport à une norme de mesure prédéfinie.

4. Dispositif (1) selon l'une des revendications précédentes, **caractérisé en ce que** l'unité de mesure (2) et les autres unités de mesure sont normalisées par rapport à un volume pulmonaire d'un sujet à examiner, ou à un flux de masse fixé.

5. Dispositif (1) selon l'une des revendications précédentes, **caractérisé en ce qu'**il est prévu un appareil de table qui comporte une unité d'analyse (14).

6. Dispositif (1) selon l'une des revendications précédentes, **caractérisé en ce que** grâce à l'unité d'analyse (14), un certain nombre de cycles d'utilisation de l'unité de mesure (2) est apte à être relevé.

**7.** Dispositif (1) selon l'une des revendications précédentes, **caractérisé en ce que** l'unité de mesure (2) comporte une unité de stockage pour stocker les données de mesure relevées, analysées ou retransmises par l'unité d'analyse (14), ou le nombre de cycles d'utilisation du dispositif (1).

**8.** Dispositif (1) selon l'une des revendications précédentes, **caractérisé en ce qu'**un signal d'avertissement est apte à être émis par le dispositif (1) lorsqu'un nombre de cycles d'utilisation de l'unité de mesure (2) réglé au préalable est atteint.

**9.** Procédé pour le fonctionnement d'un dispositif (1) destiné à mesurer la respiration pulmonaire d'un sujet à examiner et comportant une unité de mesure (2) et une unité de support (3), **caractérisé par** les étapes suivantes :

- étalonnage de l'unité de mesure (2) et d'une autre unité de mesure pour mesurer un flux de volume ou un flux de masse d'air, pour que globalement les mêmes valeurs de mesure puissent être relevées avec les deux unités de mesure ;
- fixation amovible de l'unité de mesure (2) à l'unité de support (3) ;
- mesure d'une température grâce à un capteur de température, d'une humidité grâce à un capteur d'humidité, d'une pression grâce à un capteur de pression, et d'un flux de masse grâce à un capteur de flux de masse,
- définition d'un flux de volume à partir de données de mesure des mesures du capteur de température, du capteur de pression, du capteur d'humidité et du capteur de flux de masse ; et
- remplacement de l'unité de mesure (2) par l'autre unité de mesure (2') étalonnée, quand l'unité de mesure (2) n'est plus étalonnée.

**10.** Procédé selon la revendication 9, **caractérisé en ce que** les étapes supplémentaires suivantes peuvent être réalisées entre l'étape de la liaison amovible et l'étape du remplacement ;

- relevé d'un certain nombre de cycles d'utilisation de l'unité de mesure (2) grâce à une unité d'analyse (14) ; et
- émission d'un signal d'avertissement lorsqu'un nombre de cycles d'utilisation de l'unité de mesure (2) réglé au préalable est atteint.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2388665 A
- WO 03024317 A2
- WO 0156454 A2
- US 5518002 A
- WO 9720500 A
- WO 02071017 A2